# EUROPEAN PATENT SPECIFICATION

(11) **EP 0 939 083 B1**
(45) Date of publication and mention of the grant of the patent: **10.12.2003**
(21) Application number: 98124195.3
(22) Date of filing: 21.12.1998
(51) Int. Cl.: C07K 14/47, A61K 38/17, A61P 43/00

(54) **Process for the preparation of a ferro-succinylcasein complex**
Verfahren zur Herstellung eines Ferro-Succinylcaseinkomplexes
Procédé de la production d'un complexe de ferro-succinylcaséine

(30) Priority: 24.12.1997 IT MI972875
(43) Date of publication of application: 01.09.1999
(73) Proprietor: ITALFARMACO S.p.A., 20126 Milano (IT)
(72) Inventor: Bonifacio, Fausto, 04100 Latina (IT); Massardo, Pietro, 00154 Rome (IT); Di Leo, Diego, 03013 Ferentino (Frosinone) (IT)
(74) Representative: Gervasi, Gemma, Dr.

(56) References cited:
- EP-A- 0 319 664
- EP-A- 0 419 359
- CREMOSI P. & CARAMAZZA I.: "Chemical and biological characterization of iron-protein succinylate (ITF282)" INTERNATIONAL JOURNAL OF CLINICAL PHARMACOLOGY, THERAPY AND TOXICOLOGY, vol. 31, no. 1, 1993, pages 40-51, XP001012289
- PAGELLA P.G. ET AL.: "Pharmacological and toxicological studies on an iron-succinyl-protein complex (ITF282) for oral treatment of iron deficiency anemia" ARZNEIMITTEL-FORSCHUNG/ DRUG RESEARCH, vol. 34(II), no. 9, 1984, pages 952-958, XP002172941

## Description

### Scope of invention

The present invention falls within the field of protein derivatives carrying iron to the organism. Described herein is the process for preparing a ferric complex of succinglated casein. The complex according to the present invention, which is obtained in high yields and is free from insoluble residue, presents high levels of tolerability and absence of side effects.

### State of the art

Iron plays in man and animals an irreplaceable physiological role, as it is present, as essential component of proteins, enzymes, cytochromes, etc., in all the tissues of the organism. The tissues of an adult human being contain on average from 1.5 to 5 g of iron, 60-65% of which is concentrated in the red blood cells, whilst 18-30% is bound to the proteins ferritin and haemosiderin, the remaining iron being distributed in various enzymatic systems.

The requirement of this metal (1 mg/day) is met in part through the use of endogenous iron, deriving from the breakup of red blood cells, and in part through the intake of exogenous iron found in food. This requirement often is not met, both on account of an insufficient intake in the diet, and as a consequence of malabsorption, physiological alterations due to periodic haemorrhage, infective diseases, pregnancy, breast-feeding, and imperfect metabolic utilization.

The above-mentioned physiological alterations manifest themselves pathologically with the onset of hypochromic sideropenic anaemia, the most appropriate treatment of which consists in supplying the organism with iron, which is obtained via the administration of iron carriers by oral, parenteral or intravenous route.

The intake of exogenous iron is frequently accompanied by undesired side effects, usually linked to the nature of the vehicle used for administration.

Known to the state of the art are orally administered compositions based on iron salts of organic acids (such as, citrate, cholinate, aspartate, fumarate, gluconate, glyconate, lactate, oxalate, and succinate), iron salts of inorganic acids (such as, ferric chloride, ferrous sulphate, and ferric phosphate), or polymeric vectors, such as ferro-polysaccharides.

The above compositions for oral use, albeit efficacious, are accompanied by the onset of side effects, giving rise to serious gastro-intestinal impairment, which is manifested by diarrhoea and vomiting in the most serious cases. In the case of intramuscular treatment, allergic reactions, thermal jumps, tachycardia, leukocytosis and lymphadenopathy have been encountered, whilst in the case of treatment via the intravenous route, even cases of shock of an anaphylactic type, thrombophlebitis and circulatory collapse have been found to occur.

It follows that the treatment of iron deficiency is frequently affected by the poor tolerability of the preparations, thus making continued administration of the necessary quantities of iron difficult.

In order to minimize the above-mentioned side effects, it is generally recommended that food or antacids should be taken at the same time, but this leads to a reduction in iron absorption, which depends upon the composition of the food itself.

In a search for alternative vehicling agents for iron that are free from these side effects, various proteins having a certain affinity for iron have been proposed.

These may be of animal origin (such as, sero-proteins, organ proteins, ovalbumin and lactoproteins) or of vegetable origin (such as soya proteins). However, the therapeutic effectiveness of the above-mentioned vehicles, usually obtained by reaction with ferric salts, is considerably limited by a series of negative factors, including:
- insolubility of the complexes when the quantities of iron bound to the protein vehicle exceed 0.5% and the impossibility of making a precise evaluation of the total quantity of iron actually bound to the protein as against the quantity that has co-precipitated in the form of hydrated oxides, which are extremely harmful to the gastric mucosa;
- inhomogeneity in administration and difficulty in obtaining compounds containing iron in reproducible and constant concentrations:

The above disadvantages have been partially solved by succinylating the above proteins appropriately before complexing them with iron. In this way, it has been possible to obtain ferroprotein derivatives with high iron content, characterized by a good solubility at pH values higher than 5.

The British patent application No. GB-A-2115821 describes protein-succinylated iron complexes obtained starting from powder milk or mixtures of milk proteins. However, during the preparation of these complexes, alongside the soluble derivatives of iron, a fraction of insoluble derivatives in the form of mucilage is obtained, above all when operating on an industrial scale. This mucilage is difficult to eliminate, in that the degree of its formation varies in an uncontrollable way even with minimal variations in the experimental parameters. The presence of mucilage renders it necessary to adopt particular measures of purification in order to obtain perfectly soluble products.

The formation of insoluble residue moreover reduces the yield of the desired end product.

P. Cremonesi et al. *International Journal of Clinical Pharmacology, Therapy and Toxicology*, Vol. 31 No. 1-1993 (40-51 ) disclose an iron succinyl casein complex containing 5% iron having gastrointestinal tolerability and which is useful for the treatment of iron deficiency anemia.

On account of the problems set forth above, the need is felt for having available ferric complexes which, unlike the compounds described above, may be prepared on an industrial scale with high yields and without the formation of insoluble residue. These complexes enable the administration of precise and reproducible quantities of iron, without inducing side effects.

### Summary

The subject of the present invention is a process for the preparation of a ferric complex of succinylated casein (hereinafter referred to as ferro-succinylcasein) starting from casein used for food purposes, characterized by the use of specific procedures of dilaceration and desiccation.

The process of the invention, which is easy to carry out on an industrial scale, leads to ferro-succinylcasein in high yields and without the formation of insoluble residue.

### Detailed description of invention

The subject of the present invention is a process for obtaining a protein derivative of iron, namely, a ferric complex of succinylated casein (hereinafter referred to as "ferro-succinylcasein") obtained by reaction of food-grade casein, i.e. casein used for food purposes, with succinic anhydride acid ferric chloride. By the term "food-grade casein" is meant casein obtained from milk coming from strictly controlled breeding farms. This product presents a level of contamination lower than 1.000 UFC/g for bacteria, and lower than 100 UFC/g for moulds.

The use of food-grade casein leads to obtain particularly pure products, as compared to similar complexes obtained starting from usual milk proteins.

The present process for obtaining ferro-succinylcasein by reaction of food-grade casein with succinic anhydride and ferric chloride, is characterized in that:
the succinylcasein and ferro-succylcasein obtained in the course of the process are dilacerated using dilacerating pumps at a pH of between 6 and 11; and
the ferro-succinylcasein, precipitated by acidification and recovered by filtration at the end of the process, is dried in 15-36 hours at a temperature of between 60°C and 80°C, at a pressure of between 2666,44 and 26664,4 Pa (20 and 200 mmHg; 0.0263 and 0.263 atm), keeping the temperature between 70°C and 75°C during the first 13 hours of desiccation.

Dilaceration is a process involving high mechanical energy characterized by the use of pumps, which enables elimination of aggregations of suspended solid material which cannot be eliminated by mere mechanical agitation. Dilaceration pumps are special pumps wherein the movement of a propeller is associated to the movement of a grinding gear. The suspension is forced through the pump, via the depression exherted by the rotating propeller. At the same time, the liquid is forced through the grinding gear, whereby dilaceration takes place. Dilaceration pumps are commercially available, e.g. Mouvex 20/10. It is essential that dilaceration should take place at pH values of between 6 and 11, and preferably between 8 and 9: beyond pH 11 protein degradation occurs.

As in the case of dilaceration, also drying contributes to a critical extent to obtaining ferro-succinylcasein in high yields and without insoluble residue. Drying must be carried out within 15-36 hours, preferably between 20 and 24 hours, at a temperature of between 60°C and 80°C and at a residual pressure of between 2666,44 and 26664,4Pa (20 and 200 mmHg; 0.0263 and 0.263 atm). It is moreover essential that during the first 13 hours of desiccation the temperature should be kept between 70°C and 75°C. The type of drier used is not of critical importance for the purpose of the invention.

As a whole, the process of synthesis of the ferro-succinylcasein according to the present invention comprises the following stages:
**1)** succinylation of food-grade casein with succinic anhydride, followed by precipitation and filtration of the succinylcasein obtained;
**2)** reaction of the succinylcasein with ferric chloride, precipitation and filtration of the ferric complex of succinylated casein obtained;
**3)** purification and isolation of the ferric complex of succinylated casein obtained in stage 2);
**4)** desiccation of the product obtained in stage 3).

The various stages are described in detail as follows:

In stage 1), succinylation is performed by dissolving food-grade casein in water and adding succinic anhydride at a pH of between 6 and 9, preferably of between 7.5 and 8.5. The reaction can be favoured by blowing nitrogen gas into the solution in order to prevent accumulation of succinic anhydride.

The succinylated product is precipitated via acidification at a pH value of between 1 and 4, preferably of between 2.5 and 3, and separated by filtration.

The product, collected from the filter, is suspended in water and soda and subjected to dilaceration at a pH of between 6 and 11, preferably of between 8 and 9, until a solution is obtained.

In stage 2), the reaction with ferric chloride may be performed by adding an aqueous solution of ferric chloride to the solution obtained at the end of stage 1), until a pH of between 2.5 and 3 is obtained. A suspension is formed, which is subjected to filtration, and crude ferro-succinylcasein is recovered as insoluble phase.

In stage 3), the purification is performed as follows: the crude derivative obtained in stage 2) is suspended in water and kept under stirring for approximately 2 hours.

In this stage, it is possible to add aqueous solutions of preservatives. Specific examples of useful preservatives are methyl *para*--hydroxybenzoate and propyl *para*-hydroxybenzoate. The addition of these preservatives contributes to maintaining the microbiological quality of the product, which must meet the microbiological requirements for pharmaceutical preparations.

The suspension, possibly containing the preservatives, undergoes a new dilaceration phase, carried out at a pH of between 6 and 11, preferably of between 8 and 9, which leads to the ferro-succinylcasein solution.

The aqueous phase is further clarified by filtration.

From the clarified solution, the ferro-succinylcasein derivative is precipitated by acidification and is then filtered and recovered.

In stage 4), the ferro-succinylcasein derivative undergoes desiccation at a temperature of between 60°C and 80°C, at a pressure of between 2666,44 and 26664,4 Pa (20 and 200 mmHg), for a period of time of between 15 and 36, preferably between 20 and 24 hours. It is moreover essential that during the first 13 hours of desiccation the temperature should be between 70°C and 75°C.

At the end of desiccation, the product contains a maximum moisture residue of less than 5% and a content of bound iron of between 5% and 5.6% by weight, calculated on the dry substance. For a complete characterization of the product, reference is made to the data provided in Example 1.

The process described makes it possible to obtain ferro-succinylcasein free from residue or from iron derivatives that are insoluble or poorly soluble in water. In particular, the product is found to be completely soluble at neutral/alkaline pH values, i.e., the ones typical of the intestinal tract, thus guaranteeing maximum iron bio-availability for the purpose of intestinal absorption.

The process described moreover makes it possible to obtain ferro-succinylcasein in high yields, generally between 94% and 98%. These yields are considerably higher than those obtainable according to the process described in GB-A-2115821.

The ferro-succinylcasein complexes according to the present invention have a constant composition, present excellent pharmacological activity linked to their behaviour as iron carriers, and are free from side effects. The therapeutic use of this product does not involve the disadvantages of known iron-based compounds, in particular gastric lesions.

The product according to the present invention can be adequately compounded in pharmaceutical formulations suitable for oral administration.

In order to provide an illustration of the present invention the following experimental example is given.

### Example 1 - Preparation of ferro-succinylcasein

### (A) Succinylation of casein used for food purposes

The water used in the entire process is microbiologically pure.

A stainless-steel reactor provided with anchor mixer and with a probe for reading pH values, is filled with 2700 I of depurated water and 200 Kg of casein used for food purposes, and the mixture is kept under stirring until a homogeneous suspension is obtained at pH 5. Approximately 20 I of a 15% w/v NaOH solution are added, at a temperature of 20°C, in a period of 20 minutes, until a pH value of 8 is obtained.

To the solution thus prepared, the following are added at a temperature of 20°C: 60 Kg of succinic anhydride, and approximately 150 I of a 15% NaOH solution, so as to maintain the pH value between 7.5 and 8.5. During the process, which has a duration of approximately 2 hours, at intervals nitrogen gas is blown in from the bottom of the reactor, in order to prevent accumulation of succinic anhydride.

At the end of the reaction, the mixture is left to stand for 60 minutes.

### (B) Transfer and purification of succinylated casein via precipitation

The solution obtained from stage (A) is transferred into an enamelled reactor provided with a mixer and a suitable probe for reading pH values.

The solution, kept at a temperature of 20°C, is then acidified with approximately 150 I of a 15% w/v HCl solution in a period of 45 minutes, until a pH 3 is obtained.

The decrease in the pH value causes the formation of a precipitate of succinylated casein depurated from other salts, other reaction products, and other soluble impurities.

### (C) Filtration and dilaceration

The succinylcasein obtained in stage (B) is recovered by filtration, and then the reactor and filter are washed with 100 I of depurated water. The moist product is removed from the filter, and then put into a stainless-steel reactor equipped with an anchor mixer and with a probe for reading pH values which has been previously charged with 2700 I of depurated water, and then subjected to agitation.

The suspension that forms is kept under agitation for 1.5-2 hours, at a temperature of 20°C, and then subjected to dilaceration.

After 30 minutes of dilaceration, approximately 60 l of a 15% w/v NaOH solution are added to the suspension, until a stable pH value of between 8.2 and 8.5 is obtained. At the end of the addition, the reaction product is once more treated in the same reactor, for a period of approximately 6 hours, using dilacerating pumps.

This operation facilitates the disgregation of the solid particles of succinylated casein.

### (D) Clarification, reaction with ferric chloride and filtration of crude ferro-succinylcasein

After interrupting agitation for 30 minutes, the succinylated-casein suspension is filtered. The reactor and filter are washed with 200 lit of depurated water, and the washing water is then added again to the clarified solution.

This solution is sent on to an enamelled reactor equipped with impeller mixer and with a probe for reading pH values.

A solution of 58.3 kg of ferric chloride in 600 I of water is then added at a temperature of 20°C in 40 minutes, until a stable pH value of 3 is obtained (possible corrections of the pH value may be made using 15% HCl). A suspension of ferro-succinylcasein is obtained. The reaction product is kept under agitation for 30 minutes, and the crude ferro-succinylcasein derivative is recovered by filtration.

The reactor and filter are washed with 100 l of depurated water.

### (E) Partial purification of ferro-succinylcasein and addition of preservatives

The crude ferro-succinylcasein derivative, recovered from the filter in the previous stage, is loaded into a stainless-steel reactor equipped with anchor mixer and with a special probe for reading the pH value, containing 3000 I of depurated water, and then subjected to agitation. The suspension that forms is kept under agitation for 1 hour at room temperature and subjected to the action of a dilacerating pump for 1 hour.

Approximately 30 I of a 15% NaOH aqueous solution are then added, again at a temperature of 20°C, until a pH value of between 5.5 and 6.5 is obtained.

To the reaction mixture, two solutions are then added, consisting of 13.3 Kg of methyl *para*-hydroxybenzoate in 50 I of depurated water, and 3.6 Kg of propyl *para*-hydroxybenzoate in 50 I of depurated water. At the end of the addition of the above-mentioned preservatives, the reaction product presents a pH value of 7.2.

To the suspension thus obtained are added approximately 10 I of a 15% NaOH aqueous solution, at a temperature of between 20°C and 25°C, until a stable pH value of between 8.3 and 8.5 is obtained. During this addition, the reaction product is subjected to dilaceration for 8 hours, using dilacerating pumps. A solution is obtained, which is left to stand for 1 hour.

### (F) Clarification, purification and isolation of pure moist ferro-succinylcasein

The solution obtained from the previous stage is filtered to yield an insoluble residue of less than 0.5 wt%.

To the clear solution, which is put into an enamelled reactor equipped with an impeller mixer and with a probe for reading the pH value, approximately 40 I of a 15% HCl aqueous solution are added by pouring, at a temperature of between 20°C and 25°C, in approximately 30-40 minutes, until a pH value of 2.8-3 is obtained.

At the end of the addition, the reaction product is kept stirred for 30 minutes, and the precipitate of pure ferro-succinylcasein that has formed is recovered by filtration and subsequent washing with depurated water.

### (G) Desiccation of ferro-succinylcasein

The moist pure product obtained in the previous stage is dried at a pressure of 19998,3 Pa (150 mmHg; 0.197 atm), at a temperature increasing from 60°C to 80°C, for an overall period of 24 hours, making sure to maintain the temperature between 70°C and 75°C during the first 13 hours of desiccation.

When desiccation is completed, 225 kg of pure ferro-succinylcasein are obtained, with a water content < 5 wt% (Karl Fischer), having the following chemical and chemico-physical characteristics:

| | |
|---|---|
| Colour | brown-red |
| Solubility | soluble in NaOH pH = 8 |
| pH | 2.7 |
| Free iron | < 50 ppm |
| Total iron | 5.4 wt% |
| Proteins | 76.52 wt% |
| Total succinic acid | 8 wt% |
| Free succinic acid | 0.9 wt% |
| Chlorides | 1.58 wt% |
| Methyl *para*-hydroxybenzoate | 3 wt% |
| Propyl *para*-hydroxybenzoate | 1.05 wt% |

In addition, careful analyses have confirmed the absence of microbiological contamination.

## Claims

1. Process for the preparation of ferro-succinylcasein via (a) reaction of casein with succinic anhydride, to form succinylcasein, and (b) reaction of the succinylcasein with ferric chloride, to form ferro-succinylcasein, **characterised in that**:
the casein utilised is "food grade" casein;
the succinylcasein and ferro-succylcasein obtained in the course of the process are dilacerated using dilacerating pumps at a pH of between 6 and 11; and
the ferro-succinylcasein, precipitated by acidification and recovered by filtration at the end of the process, is dried in 15-36 hours at a temperature of between 60°C and 80°C, at a pressure of between 2666,44 and 26664,4 Pa (20 and 200 mmHg; 0.0263 and 0.263 atm), keeping the temperature between 70°C and 75°C during the first 13 hours of desiccation.

2. Process according to claim 1, in which the phases of dilaceration are conducted at a pH of between 8 and 9.

3. Process according to Claim 1, in which the phase of desiccation is conducted in a time interval of between 20 and 24 hours.

4. Process according to claim 1, in which in stage (a) the reaction of casein with succinic anhydride is performed by dissolving food-grade casein in water and adding succinic anhydride at a pH of between 6 and 9.

5. Process according to claims 1 to 4, in which the succinylcasein formed in stage (a) is precipitated via acidification at a pH value of between 1 and 4, separated by filtration, suspended in water and soda and subjected to dilaceration until a solution is obtained.

6. Process according to claims 1 to 5, in which in stage (b) the reaction of the succinylcasein with ferric chloride is performed by adding an aqueous solution of ferric chloride to the dilacerated solution of succinylcasein obtained at the end of stage (a), until a pH of between 2.5 and 3 is obtained.

7. Process according to claims 1 to 6, in which the crude ferro-succinylcasein formed in stage (b) is recovered by filtration and suspended in water; the suspension undergoes a new dilaceration phase to obtain the ferro-succinylcasein solution which is clarified by filtration.

## Patentansprüche

1. Verfahren zur Herstellung von Ferro-Succinylcasein über (a) die Reaktion von Casein mit Bernsteinsäureanhydrid zur Bildung von Succinylcasein und über (b)die Reaktion von Succinylcasein mit Eisenchlorid zur Bildung von Ferro-Succinylcasein **dadurch gekennzeichnet, dass**:
• das eingesetzte Casein "Lebensmittel-Reinheitsgrad" besitzt;
• das Succinylcasein und das Ferro-Succinylcasein, die im Laufe des Verfahrens gewonnen werden, mithilfe von Dilazerationspumpen bei einem pH-Wert zwischen 6 und 11 zerrissen werden; und
• das Ferro-Succinylcasein, das durch Ansäuerung präzipitiert (ausgefällt) und am Ende des Prozesses durch Filtration wiedergewonnen wurde, für 15 - 36 Stunden bei einer Temperatur von 60 - 80 °C und einem Druck zwischen 2666.44 und 26664.4 Pa (20 und 200 mg Hg; 0,0263 und 0,263 atm) getrocknet wird unter Aufrechterhaltung einer Temperatur zwischen 70 °C und 75 °C während der ersten 13 Stunden der Trocknung.

2. Verfahren gemäß Anspruch 1, bei dem die Phasen der Dilazeration bei einem pH-Wert zwischen 8 und 9 ausgeführt werden.

3. Verfahren gemäß Anspruch 1, bei dem die Phase der Trocknung während eines Zeitintervalls von 20 bis 24 Stunden ausgeführt wird.

4. Verfahren gemäß Anspruch 1, bei dem in Stufe (a) die Reaktion von Casein mit Bernsteinsäureanhydrid durch Auflösen des Caseins mit Lebensmittel-Reinheitsgrad in Wasser und Zugabe von Bernsteinsäureanhydrid bei einem pH-Wert zwischen 6 und 9 durchgeführt wird.

5. Verfahren gemäß den Ansprüchen 1 bis 4, bei dem das in Stufe (a) gebildete Succinylcasein durch Ansäuerung bei einem pH-Wert von 1 präzipitiert, durch Filtration abgetrennt, danach in Wasser und Soda suspendiert und anschließend einer Dilazeration unterworfen wird, bis eine Lösung entsteht.

6. Verfahren gemäß den Ansprüchen 1 bis 5, bei dem in Stufe (b) die Reaktion von Succinylcasein mit Eisenchlorid ausgeführt wird durch Zugabe einer wässrigen Lösung von Eisenchlorid zu der am Ende von Stufe (a) erhaltenen dilazerierten Lösung von Succinylcasein, bis ein pH-Wert zwischen 2,5 und 3 erreicht ist.

7. Verfahren gemäß den Ansprüchen 1 bis 6, bei dem das unbehandelte Ferro-Succinylcasein, das in Stufe (b) gebildet wurde, durch Filtration wiedergewonnen und in Wasser suspendiert wird; die Suspension wird einer erneuten Dilazerationsphase unterzogen, um so eine Lösung von Ferro-Succinylcasein zu erhalten, die durch Filtration geklärt wird.

## Revendications

1. Procédé pour la préparation de ferro-succinylcaséine via (a) la réaction de caséine avec de l'anhydride succinique, pour former de la succinylcaséine, et (b) la réaction de la succinylcaséine avec du chlorure ferrique, pour former de la ferro-succinylcaséine, **caractérisé en ce que** :
la caséine utilisée est de la caséine "de qualité alimentaire" ;
la succinylcaséine et la ferro-succinylcaséine obtenues au cours du procédé sont dilacérées en utilisant des pompes de dilacération à un pH entre 6 et 11 ; et
la ferro-succinylcaséine, précipitée par acidification et récupérée par filtration à la fin du procédé, est séchée en 15-36 heures à une température entre 60°C et 80°C, à une pression entre 2666,44 et 26664,4 Pa (20 et 200 mmHg ; 0,0063 et 0,263 atm), en maintenant la température entre 70°C et 75°C pendant les 13 premières heures de dessiccation.

2. Procédé selon la revendication 1, dans lequel les phases de dilacération sont entreprises à un pH entre 8 et 9.

3. Procédé selon la revendication 1, dans lequel la phase de dessiccation est entreprise pendant un intervalle de temps compris entre 20 et 24 heures.

4. Procédé selon la revendication 1, dans lequel, au stade (a), la réaction de la caséine avec l'anhydride succinique est accomplie en dissolvant la caséine de qualité alimentaire dans l'eau et en ajoutant de l'anhydride succinique à un pH entre 6 et 9.

5. Procédé selon les revendications 1 à 4, dans lequel la succinylcaséine formée au stade (a) est précipitée via acidification à une valeur de pH entre 1 et 4, séparée par filtration, mise en suspension dans l'eau et de la soude et soumise à une dilacération jusqu'à ce que l'on obtienne une solution.

6. Procédé selon les revendications 1 à 5, dans lequel au stade (b), la réaction de la succinylcaséine avec du chlorure ferrique est accomplie en ajoutant une solution aqueuse de chlorure ferrique à la solution dilacérée de succinylcaséine obtenue à la fin du stade (a) jusqu'à ce qu'un pH entre 2,5 et 3 soit obtenu.

7. Procédé selon les revendications 1 à 6, dans lequel la ferro-succinylcaséine brute formée au stade (b) est récupérée par filtration et mise en suspension dans l'eau ; la suspension subit une nouvelle phase de dilacération pour obtenir la solution de ferro-succinylcaséine qui est clarifiée par filtration.
